# EUROPEAN PATENT SPECIFICATION

(11) **EP 2 672 257 B1**
(45) Date of publication and mention of the grant of the patent: **08.05.2019**
(21) Application number: 13183326.1
(22) Date of filing: 08.03.2005
(51) Int. Cl.: G01N 21/64, G01N 21/76, H01L 31/0352, H01L 31/09, H01L 31/102, H01L 31/08, G01N 27/414

(54) **Nanowire light sensor and kit with the same**
Nanodraht-Lichtsensor und Kit damit
Capteur de lumière à nanofil et kit le comprenant

(30) Priority: 08.03.2004 KR 20040015629
(43) Date of publication of application: 11.12.2013
(62) Divisional of application: 05726939.1
(73) Proprietor: Korea Institute of Science and Technology, Seoul 136-791 (KR)
(72) Inventor: Pyun, Jae-Chul, 66123 Saarbrücken (DE); Choi, Heon-Jin, 136-060 Seoul (KR)
(74) Representative: advotec.

(56) References cited:
- WO-A1-02/080280
- WO-A2-02/48701
- WO-A2-03/005450
- WO-A2-2004/004927
- US-A1- 2004 043 527
- ZHANG DAIHUA ET AL: "Doping dependent NH3 sensing of indium oxide nanowires", APPLIED PHYSICS LETTERS, AIP, AMERICAN INSTITUTE OF PHYSICS, MELVILLE, NY, US, vol. 83, no. 9, 1 September 2003 (2003-09-01), pages 1845-1847, XP012035970, ISSN: 0003-6951, DOI: 10.1063/1.1604194
- LI CHAO ET AL: "In2O3 nanowires as chemical sensors", APPLIED PHYSICS LETTERS, AIP, AMERICAN INSTITUTE OF PHYSICS, MELVILLE, NY, US, vol. 82, no. 10, 10 March 2003 (2003-03-10), pages 1613-1615, XP012033496, ISSN: 0003-6951, DOI: 10.1063/1.1559438
- KANG B ET AL: "pH measurements with single ZnO nanorods integrated with a microchannel", APPLIED PHYSICS LETTERS, AIP, AMERICAN INSTITUTE OF PHYSICS, MELVILLE, NY, US, vol. 86, no. 11, 8 March 2005 (2005-03-08) , pages 112105-112105, XP012064615, ISSN: 0003-6951, DOI: 10.1063/1.1883330

## Description

### BACKGROUND OF THE INVENTION

### 1. Field of the Invention

The present invention relates to a method for manufacturing a nanowire light sensor.

### 2. Description of the Background Art

A rapid test kit for immunoassay (hereinafter called as "Rapid test kit") is a point-of-care test kit for test examination using body fluids of a general person. Home pregnancy test kits, AIDS test kits for emergency rooms or the like are typical rapid test kits.

A rapid test kit generally being used has a strip type structure. A liquid sample injected into a strip moves according to a capillary phenomenon. A basic principle of such a rapid test kit is immunoassay, in which an analyte in the sample generates an antigen-antibody reaction at a reaction point located within a moving path and a result of such a reaction is checked with the naked eye through chemical color development.

Effectiveness determination of the rapid test kit is performed by observing reactions of the sample occurring at a negative reaction point and a positive reaction point located at the front and back of the reaction point. In addition, the checking of a normal movement of the sample and effectiveness of the test kit are performed in a test process by a macroscopic examination through the chemical color development. Such a rapid test kit corresponds to a limited quantitative analysis which checks that the analyte in the sample exists above a certain concentration. A method for locating a plurality of reaction points is applied for subdivided quantitative analysis .

A color development reaction generally being used uses Horseradish Peoxidase (HRP) bonded to an antigen or an antibody used for detection of the analyte, Alkaline Phosphatase (AP), and particular substrates with respect to these enzymes. An insoluble compound which is colorless before a reaction and causes color development of a specific color after the reaction is generally used as a specific substrate with respect to these protein.

Chemical color development used in the existing rapid test kit has a level of color development which is determined according to the amount of the analyte in the sample and can be checked with the naked eye with respect to concentration which is above a certain level. It is known that a limit of concentration of the analyte which can be checked with such color development reaction is 10⁻⁶ to 10⁻⁹ M.

Compared to this, it is known that a limit to which analysis is possible can be raised up to 10-19 M when chemiluminescence is used and up to 10⁻¹² M when chemifluorescence is used [refer to K. Dyke, Light Probes, in Luminescence biotechnology, CRC Press, 2002, pp. 5]. To construct a kit using chemiluminescence, HRP and AP used in the rapid test kit using the color development can be also used and a specific substrate generating chemiluminescence substitutes for the specific substrate generating chemical color development. In case of the chemifluorescence, a test is performed using a reactant bonded with a fluorescent material. Like this, if the chemiluminescence or chemifluorescence is used in the rapid test kit, an extremely small amount of the analyte included in various kinds of body fluid samples can be analyzed because of improved sensitivity. Thus, an application arrange of the rapid test kit can be expanded and a chemiluminescence or chemifluorescence kit can be constructed by replacing only a reaction substrate or bonding a fluorescent material. Accordingly, the existing production fatalities can be used.

When using the chemiluminescence or chemifluorescence is used, unlike the chemical color development, a test result cannot be discriminated with the naked eye. In order to check the test result, a separate detector for measuring the amount of chemiluminescence and the amount of chemifluorescence is needed. A PMT (photomultiplier tube) and a CCD (Charge-coupled device) are typical as the existing detector for measuring chemiluminescence. [refer to K. Dyke, Instrumentation for the Measurement of Luminescence, in Luminescence biotechnology, CRC Press, 2002, pp, 31-39]. However, the PMT cannot be used as disposables because it is difficult to miniaturize the PMT, and the unit cost of production is high. In addition, miniaturization of the CCD is easy but it is difficult to use the CCD without external equipment on the spot because a darkroom condition is necessary for the precise measurement because of a wide range of inductive light wavelength. Accordingly, in order to introduce the chemiluminescence in the rapid test kit, required is a detector which can be miniaturized to be embedded in a kit, whose unit cost of production is low and which can be used on the spot without separate external equipment or without blocking of light.

Zhang Daihua et al., Appl. Phys. Lett., vol. 83, no. 9 (2003-09-01) pg. 1845 - 1847 (XP012035970) or WO03/005450 disclose a nanowire sensor.

### SUMMARY OF THE INVENTION

The invention provides a method according to claim 1.

### BRIEF DESCRIPTION OF THE DRAWINGS

The accompanying drawings, which are included to provide a further understanding of the invention and are incorporated in and constitute a part of this specification, illustrate examples of the invention and together with the description serve to explain the principles of the invention.

In the drawings:
Figure 1 is a graph showing a flow of an electric current when a ZnO nanowire is connected to electrodes and light with a wavelength of 356nm and light with a wavelength of 532nm are alternately irradiated;
Figure 2 is a graph showing a current flowing on the nanowire according to pumping power;
Figure 3 is graph showing a comparison between an electric current penetrating through the nanowire by light irradiation and dark current;
Figure 4 shows a change of a bandgap which is observed when a GaN nanowire is doped with a very small amount of material;
Figure 5A shows a GaN-ZnO radial heterostructure nanowire, and Figure 5B shows a change of a bandgap which occurs in the heterostructure nanowire;
Figure 6 shows a structure of the nanowire light sensor;
Figure 7 shows a rapid test kit structure to which the nanowire light sensor is attached;
Figure 8 shows a InN nanowire grown on the substrate; and [(4): check line, (5): test line, (6) test kit strip, (7) nanowire optical switch]
Figure 9 shows a change of an electric current penetrating through the nanowire by chemiluminescence in the test kit using the InN nanowire.

### DETAILED DESCRIPTION OF THE PREFERRED EMBODIMENTS

Reference will now be made in detail to the preferred embodiments of the present invention, examples of which are illustrated in the accompanying drawings.

The present invention relates to a method for manufacturing a nanowire light sensor, and to a nanowire light sensor manufacturing using the method.

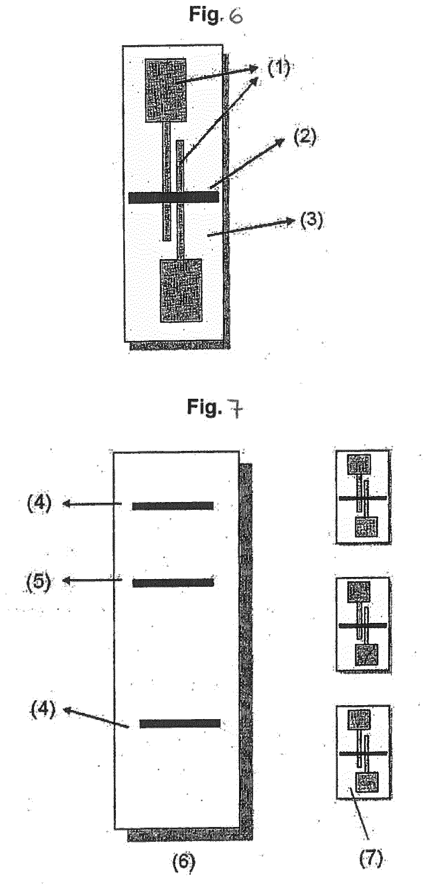

First, the present invention provides a nanowire light sensor manufacturing method which is discussed below, the nanowire light sensor including a substrate, a power supply, two electrodes connected with the power supply and a semiconductor nanowire connected to the two electrodes. As to one embodiment, a nanowire light sensor has a structure in Figure 6. As shown in Figure 6, two conductive metal thin films are located on a nonconducting substrate to thereby construct electrodes and a nanowire is coupled between the two electrodes, thereby constructing a nanowire light sensor.

The substrate is a nonconducting substrate such as a semiconductor like silicon, ceramic like sapphire, glass, a polymer, plastic or the like. Preferably, the power supply is in the range of 1 to 3 V. Preferably, the electrodes are selected from a group consisting of Au, Ti, Pt, Pd and TiN and alloys composed of two or more of them. A distance between the electrodes is controlled in the range of 2 to 100 micrometers, which is shorter than the length of the nanowire.

'Nanowire' in the present invention means a structure that a diameter is in the range of one nanometer to 100 nanometers and that the length-diameter ratio is very large. 'Semiconductor nanowire' means a structure that comprises a semiconductor material and has the above-described shape. The semiconductor nanowire can be formed of all typical semiconductor materials and preferably can be formed of one or more materials selected from a group consisting of ZnO, SnO₂, CdSe, GaN, CdS, InP, GaP, GaAs, AlAs, InN, Si, Ge and SiC.

Since the nanowire is single crystal compared to other materials, the nanowire is hardly affected by a defect or physical and chemical characteristics of impurities. In addition, a function of the nanowire as a sensor is excellent because the surface area is greatly large and a reaction and sensing force are great with respect to a change in physical and chemical environments because of an effect obtained by the nano size. In addition, since the nanowire is long enough in one direction, it is easy to manufacture the nanowire in the form of a device in comparison to another nano materials.

Meanwhile, the semiconductor is known to have a characteristic that electric resistance is reduced by light-excited at a specific wavelength (light sensing bandgap). A research to use the semiconductor nanowire as optical switches or photodetectors with respect to a specific wavelength by using this characteristic has been reported. For example, photodetectors using Zno nanowire has been reported [refer to H. Kind, H. Yan, B. Messer, M. Law, P. Yang. Nanowire ultraviolet photodetectors and optical switches, ADV. Mater. 14 (2002) 158-160]. At this time, by measuring electric resistance of the nanowire or current flowing thereon, it can be determined whether or not the specific wavelength is emitted or not and a degree of emission.

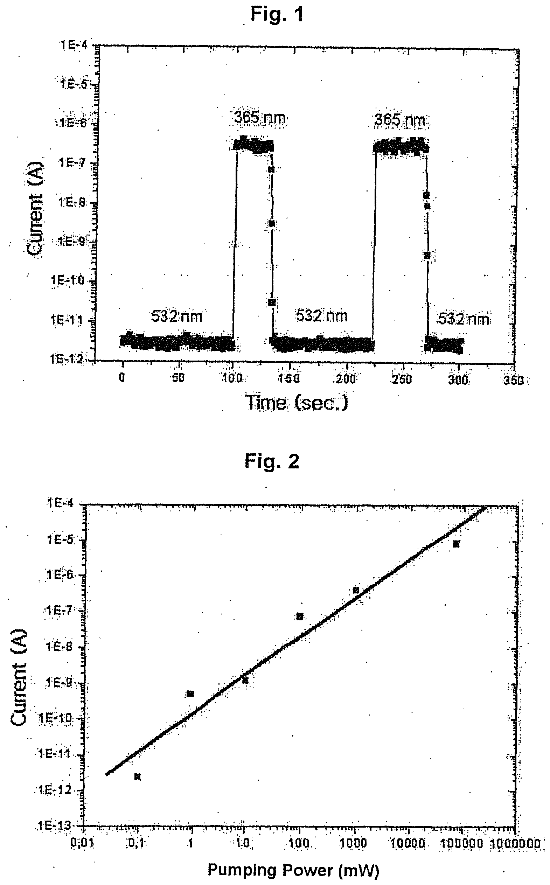

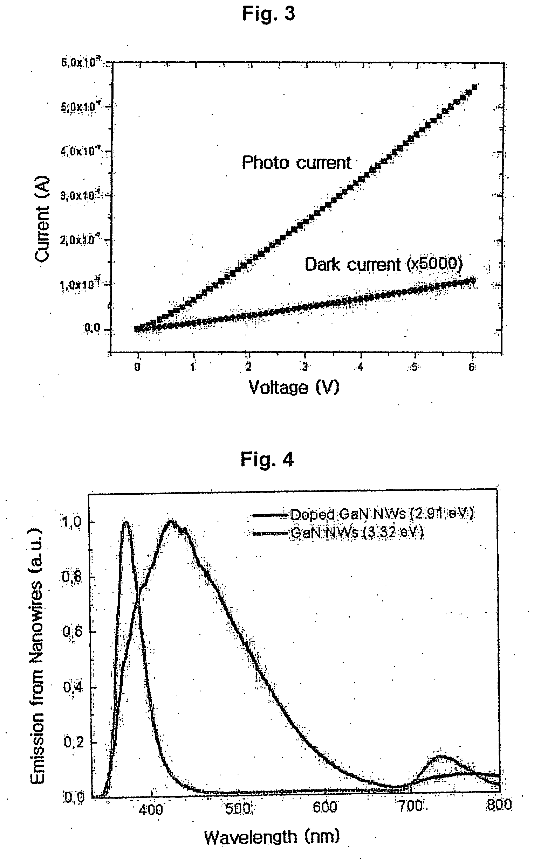

Figure 1 is a graph showing a flow of an electric current when a ZnO nanowire is connected to electrodes and light with a wavelength of 653nm and light with a wavelength of 532nm are alternately irradiated, in which electric resistance of the ZnO nanowire changes greatly when the light with a wavelength of 653nm is irradiated. Such a change in electric resistance of the nanowire with respect to the specific wavelength shows the quantitative characteristic with respect to the amount of irradiated light as shown in Figure 2 which shows the current flowing on the nanowire according to irradiation dosage (excitation power). Figure 3 is a graph showing a comparison between an electric current penetrating through the nanowire by light irradiation and dark current. As shown therein, since the electric current flowing on the nanowire by irradiated light applied on the nanowire shows a significantly high numeral value compared to dark current acting as an interference factor, a high signal to noise ratio can be secured.

In the present invention, in order that the nanowire light sensor is controlled to generate light sensitivity at a desired bandgap, a nanowire having the following characteristic can be used:

First, since nanowire has different sensitivity wavelengths according to the constructing materials, the nanowire light sensor can generate light sensitivity at a desired bandgap by selecting appropriate constructing materials. At this time, by using two or more nanowires having different sensitivity wavelengths, a sensitivity wavelength of the nanowire light sensor can be controlled. The sensitivity wavelength can be controlled by a method of making a plurality of nanowire suspensions having different sensitivity wavelengths mixed liquor and arranging it on the light sensor substrate. A bandgap with respect to the constructing materials used in the embodiment of the present invention is shown in the following Table 1.

**[Table 1]**

| Bandgap | Materials of nanowire |
|---|---|
| ≤ 360 nm | ZnO, GaN, SiC, SnO₂ |
| 360 nm to 500 nm | doped ZnO, doped GaN, doped SiC |
| 500 nm to 600 nm | AlAs, InN, CdS |
| 600 nm to 800 nm | InP, GaP, GaAs, InN, Si, CdSe |
| ≥ 800 nm | Si, Ge |

Second, by using a nanowire obtained by doping the existing nanowire with appropriate impurities, the nanowire light sensor can be controlled to generate light sensitivity at a desired bandgap. The impurities can serve as a donor and an acceptor, and about 0.5 to 5 % of the impurities are added. For instance, in case of a GaN nanowire, Si or Ge may be used as the donor and Mg, Mn, Co or Fe may be used as the acceptor. In case of a SiC nanowire, N may be used as the donor and B or Al may be used as the acceptor. In case of a ZnO nanowire, Si or Al may be used as the donor and N or Li may be used as the acceptor. In case of a Si or Ge nanowire, Li, P, As, Sb or S may be used as the donor and B, Al, Zn, In, Ga or Ni may be used as the acceptor. In case of a Inp or GaP nanowire, Si, N or As may be used as the donor and Mg, Mn or Zn may be used as the acceptor. In case of a AlAs or GaAs nanowire, Si may be used as the donor and Mg, Mn or Zn may be used as the acceptor. In case of a InN nanowire, Si or Ge may be used as the donor and Mg, Mn, Zn or the like may be used as the acceptor. In case of a CdS or CdSe nanowire, Si or Ge may be used as the donor and Mg may be used as the acceptor.

Figure 4 shows a change of a bandgap which is observed when a GaN nanowire is doped with a very small amount of material (Mn, 4%). As shown therein, a bandgap can be controlled by doping a very small amount of impurities and a bandgap in which the nanowire used in the light sensor senses can be controlled by controlling kinds and doped amount of the impurities.

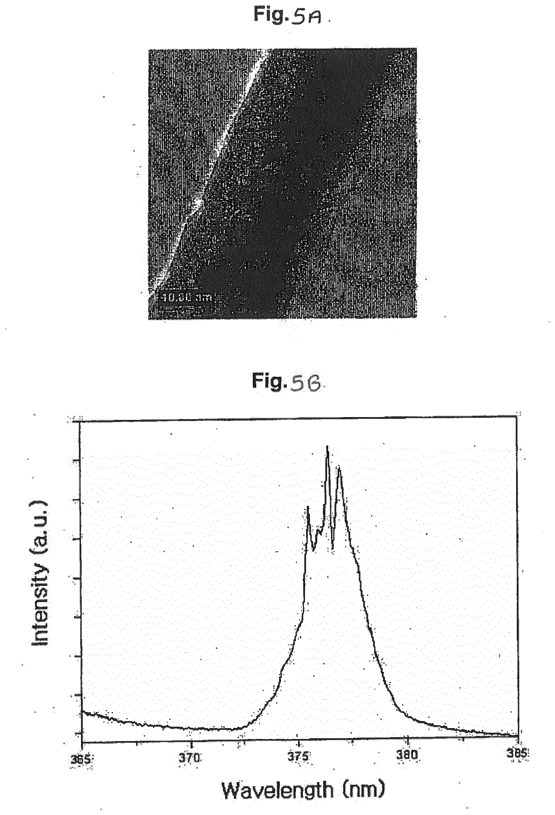

Third, by using a nanowire having radial heterostructure, the nanowire light sensor is controlled to generate light sensitivity in a desired bandgap. The radial heterostructure can be obtained by depositing one material and forming core part, and then depositing another material around the core part and forming sheath part. For example, Figure 5A shows an appearance of a GaN-ZnO radial heterostructure nanowire, and Figure 5B shows a change of a bandgap which occurs in the heterostructure nanowire. As shown in Figures 5A and 5B, since the core and the sheath can generate light sensitivities in different bandgaps, respectively, in the heterostructure nanowire where two kinds of materials construct core and sheath, respectively, a light reaction can be achieved in a wider range. In addition, a bandgap in which light sensitivity is generated can be controlled by adjusting the thickness of the core and the sheath by a quantum limit effect. That is, when the thickness of the core and the sheath is adjusted to be smaller than the Bohr exiton radius each semiconductor has, a light sensitivity bandgap can be controlled. For example, in case of GaN-ZnO heterostructure, the thickness of each part or one part is controlled to be less than 10 nm, light sensitivity can be generated in a shorter bandgap by the quantum limit effect.

Fourth, by using a nanowire having longitudinal heterostructure, the nanowire light sensor can be controlled to generate light sensitivity in a desired bandgap. The longitudinal heterostructure can be obtained by alternately depositing two or more different materials. Like this, the heterostructure nanowire in which two kinds of materials are alternately formed can achieve light sensitivity in a wider bandgap because each area of the nanowire can generate light sensitivity in each different bandgap. For example, a nanowire which has the longitudinal heterostructure can be manufactured with InN and GaN which are the same III-V compound semiconductor and have the same crystal structure. In such a nanowire, InN part generates light sensitivity in a bandgap of 500 to 800nm and GaN part generates light sensitivity in a bandgap less than 360nm.

Fifth, by using a tubular nanowire whose inside is removed from the radial heterostructure, the nanowire light sensor can be controlled to generate light sensitivity in a desired bandgap. In this case, the light sensitivity bandgap can be controlled according to the thickness of a tube by the quantum limit effect. The core part which is weak in high-temperature reduction atmosphere is removed by processing the core part with hydrochloric acid or processing the core part under the high-temperature reduction atmosphere (e.g., H₂, 500°C, 30 minutes). That is, by controlling the thickness of the remaining tube after removing the core part, the light sensitivity bandgap can be controlled. For example, when the thickness of the tube is controlled to be smaller than the Bohr exiton radius each semiconductor has, light sensitivity can be generated in a shorter bandgap by the quantum limit effect.

Sixth, by using a nanowire in a solid solution form, the nanowire light sensor can be controlled to generate light sensitivity in a desired bandgap. For example, InN and GaN of III-V compound have 0.7eV and 3.4 eV bandgaps, respectively, and can make InxGa10xN composition where InN and GaN are in the solid solution form with respect to each other. At this time, since the bandgap can be controlled by x, the light reaction can be controlled in a wider range of from 620 to 360nm according to the control of x.

The present invention provides a method of manufacturing the nanowire light sensor having such a construction. The method of manufacturing the nanowire light sensor comprises: growing a nanowire and then separating the nanowire; locating two conductive metal thin films on a non-conducting substrate; locating the nanowire between two electrodes by dispersing the obtained nanowire between the electrodes and locating the nanowire therebetween by applying 5 to 50 V range of voltages thereto; and electrically connecting the electrodes and the nanowire by electron-beam irradiation. When the electron beam is used in connecting the electrodes and the nanowire, the electron beam is irradiated for one to five seconds, preferably.

A material selected from a group consisting of Au, Ti, Pt, Pd and TiN and alloys composed of two or more of them is used as the electrode, preferably. The two electrodes composed of identical or different materials are located on the non-conducting substrate at an appropriate distance. Preferably, a distance between the two electrodes is controlled in the range of 2 to 100 micrometers, which is shorter than the length of the nanowire. Preferably, the nanowire is formed of a typical semiconductor material or preferably, one material selected from a group consisting of ZnO, SnO₂, CdSe, GaN, CdS, InP, GaP, GaAs, AlAs, InN, Si, Ge and SiC and has a diameter in the range of one nanometer to 100 nanometers.

A noble metal or transition metal catalyst is located on a predetermined substrate selected from semiconductor like silicon, ceramic like sapphire, glass, polymer and plastic substrates, a predetermined precursor of a nanowire ingredient is provided and grown at a high temperature in the range of 300°C to 800°C, and then the nanowire is separated from the substrate and used. Au, Ni, Co or Ni can be used as the transition metal catalyst. The substrate where the nanowire is grown is put in alcohol or an organic solvent which does not react to the nanowire and is easily volatilized and supersonic waves are applied for few seconds, so that the nanowire is separated from the substrate. Accordingly, a solution in a suspension state which includes the nanowire can be made and used. Isopropyl alcohol can be used as the organic solvent.

A material having a desired light sensitivity range as the nanowire is selected and grown. As described, the nanowire light sensor can be controlled to generate light sensitivity in a desired bandgap by doping a nanowire with appropriate impurities, manufacturing a nanowire having radial heterostructure or longitudinal heterostructure by using two different materials, manufacturing a tubular nanowire or manufacturing a nanowire in a solid solution form.

According to one embodiment of the present invention, the nanowire can be located between two electrodes by locating a small amount of the nanowire solvent between two electrodes located on the non-conducting substrate and applying an electric field thereto. According to another embodiment of the present invention, the nanowire solvent is made in the form of Langmuir-Blodgett film and compressed such that it can be located between the two electrodes. Or, the nanowire can be located between the two electrodes by using a predetermined method which is typically used in the technical field the present invention pertains to.

Thereafter, the electrodes are electrically connected to the nanowire by electron-beam irradiation. Whether the nanowire is electrically connected or not is determined by sequentially performing a macroscopic examination using an optical microscope and an electric test method, in which a specific wavelength is irradiated and then a change in resistance is observed. At this time, the measurement is performed applying a certain voltage to the two electrodes and the amount of irradiated light is converted by measuring the amount of an electric current flowing on the nanowire according to a resistance change of the nanowire.

In addition, the method for manufacturing the nanowire light sensor can be used when providing a detection strip including a chemifluorescence material and the nanowire light sensor manufactured by the above-described method, and providing a chemifluorescence measuring kit where the nanowire light sensor is attached to and located at part where chemifluorescence is generated (refer to Figure 7). The chemifluorescence measuring kit can be used as a rapid test kit.

As shown in Figure 7, the light sensor using such a nanowire is located and attached to a rapid test kit where chemifluorescence is performed. A fluorescent material which has a fluorescence wavelength appropriate for a wavelength at which the nanowire can sense is selected from fluorescent materials generally being used, and this is coated on at least one part (e.g., signal line and check line) of the detection strip. Each excitation wavelength according to each fluorescent material used for such measurement of fluorescence and each nanowire used with respect to each fluorescence wavelength are shown in Table 2.

**[Table 2]**

| Fluorescent material | Excitation wavelength (nm) | Fluorescence wavelength (nm) | Type of nanowire |
|---|---|---|---|
| Fluorecein | 495 | 520 | AlAs, InN |
| Biodipy-FL | 503 | 512 | AlAs, InN |
| Alexa Fluor Green | 491 | 515 | AlAs, InN |
| R-phycoerythrin | 564 | 576 | AlAs, InN |
| Phycoerythrin-Texas Red | 495 | 620 | GaP, InN |
| Phycoerythrin-cyanine5 | 495 | 670 | InP, GaP, InN |
| Phycoerythrin-cyanine7 | 495 | 755 | InP, GaP, GaAs, InN, Si |
| Peridinin-chlorophyll protein | 490 | 677 | InP, GaP, GaAs, INN |
| Allophycocyanin | 650 | 660 | GaP, GaAs |
| Allophycocyanin-cyanine 7 | 650 | 755 | InP, GaP, GaAs, Si |

As shown in Table 2, a nanowire which can react in a given bandgap was selected from a InP, GaP, GaAs, AlAs, InN, or Si nanowire in a bandgap of 500 to 800 nm. Or, radial heterostructure nanowire appropriate for the given bandgap may be selected. Or a longitudinal heterostructure nanowire appropriate for the given bandgap may be selected. Or a nanotube appropriate for the given bandgap may be selected. Or a nanowire in a solid solution form which is appropriate for the given bandgap may be selected. Like this, a nanowire used in the light sensor is appropriately selected according to a fluorescence wavelength a fluorescent material shows.

In addition, the method for manufacturing the nanowire light sensor can be used when providing a detection strip including a chemiluminescence substrate and a chemiluminescence enzyme and the nanowire light sensor, and providing a chemiluminescence measuring kit where the nanowire light sensor is attached to and located at part where chemiluminescence is generated (refer to Figure 7). The chemiluminescence measuring kit can be used as a rapid test kit.

As shown in Figure 7, the light sensor using such a nanowire is located and attached to a rapid test kit where chemiluminescence is performed (e.g., signal line and check line). A luminous material which has a luminescence wavelength appropriate for a wavelength at which the nanowire can sense is selected from luminous substrates generally being used, and this is coated on at least one part of the detection strip to thereby be used as a detection target substrate. Chemiluminescence is generated by injecting the chemiluminescence enzyme acting on the detection substrate and then measured. For example, the detection strip is coated with adamantane-dioxetane, an acridinium derivative, a luminol derivative, lucigenin, firefly luciferin, photoprotein including aequorin or the like, hydrazides and schiff basic compounds, an electrochemical luminous substrate including a ruthenium trisbipyridyl group or a luminous oxide channeling substrate which generates quantitative chemiluminescence in a bandgap shown in Figure 2, among luminescence substrates of horseraddish peroxidase (HRP), alkaline phosphatase (AP) and luciferase, and therefore can be used as the detection target substrate. Each luminescence wavelength according to each luminous substrate and each nanowire sensing at each wavelength are shown in Table 3.

**[Table 3]**

| luminous substrate | luminescence wavelength | Type of nanowire |
|---|---|---|
| Adamatane-dioxane (dioxetane) | 477 | GaP, AlAs |
| Acridinium compounds | 430-435 | Doped GaN, doped ZnO |
| Luminol compounds | 500 | GaP, AlAs, InN |
| Luciferin | 565 | InP, GaAs |
| Photoprotein | 470 | InP, GaP, GaAs, AlAs, InN |
| Hydrazides and Shiff basic compounds | 540 | InP, GaP, GaAs, AlAs |
| electrochemical luminous substrate | 620 | InP, GaAs, AlAs, Si, Ge |
| luminous oxide channeling substrate | 520-620 | InP, GaAs, AlAs |

As shown in Figure 3, a doped GaN or doped ZnO nanowire is used as a nanowire in a 430 nm bandgap. In a bandgap longer than 430 nm, a InP, GaP, GaAs, AlAs, InN, Si or Ge nanowire is appropriately selected and used. A radial heterostructure nanowire having a core and sheath structure may be used by selecting two materials in order to conform to a given bandgap. A longitudinal heterostructure nanowire obtained by alternately depositing two materials selected to conform to a given bandgap may be used. A nanowire where two materials are appropriately in a solid solution form may be used. Like this, a nanowire used in the light sensor is used according to a luminescence wavelength a luminous substrate shows.

A light sensor using a nanowire can be easily miniaturized to be embedded in a rapid test kit, has a low unit cost of production, and can be used on the spot without separate external equipment. In addition, an optical switch using the nanowire can be embedded in a plastic container for a rapid test kit without a separate light blocking device because a bandgap of light in which the nanowire senses is narrow unlike a typical CCD device or a light diode. Also, the optical switch using the nanowire can be operated using a button type battery which can be embedded in the rapid test kit because it has low power consumption.

In addition, the method for manufacturing the nanowire light sensor can be used when providing an immunoassay kit including the nanowire light sensor and an analyte. The immunoassay kit includes a nucleic acid detection chip where the analyte is nucleic acid including oligonucleotide, DNA, RNA, PNA and cDNA and a protein chip where the analyte is protein.

First, the nanowire light sensor can be applied to the existing nucleic acid detection chip and the protein chip which use a fluoresce reaction. When a gene is detected using the nucleic acid chip, in order to check whether a complementary gene is bonded to a gene fixed to the chip or not, a fluorescent material is connected to the complementarily bonded gene, where fluorescence being generated is measured in general. In effect, the fluorescent is checked by irradiating a laser with a specific wavelength to generate fluorescence and measuring the amount of light with a shifted wavelength. The measurement of fluorescence is performed at the 90 ° angle to a light path of a laser generally being used as a light source. At this time, the nanowire light sensor which uses a nanowire having a bandgap appropriate for a fluorescence wavelength of the fluorescent material used at the 90 ° angle to the light source is provided and therefore the fluorescence being generated can be measured.

In addition, the nanowire light sensor capable of measuring chemiluminescence can be applied to the nucleic acid detection chip or the protein chip. To do so, the complementary gene bonded to the gene fixed to the chip is bonded to an enzyme such as HRP, AP, luciferase or the like which can induce chemiluminescence and a chemiluminescence substrate is injected thereinto, so that the detection can be performed. A limit to which the detection is possible with fluorescence is 10⁻¹²M in general, while a limit to which the detection is possible with chemiluminescence is 10⁻¹⁹M. When the chemiluminescence is used, high-sensitivity detection is possible in comparison to chemifluorescence. Accordingly, since a target gene is not amplified using a PCR method or a small amount of the analyte gene can be detected with amplification performed only a few times, test time is reduced and detection errors caused by wrong amplification can be decreased. In addition, since a separate light source such as a laser is not needed, it is easy to miniaturize a micro scale, and since the nanowire light sensors of the present invention can be integrated to have an array form, the nanowire light sensor has an advantage in an application to the nucleic detection chip.

In addition, as to the protein chip, protein is fixed and a bonding material comprising protein or a chemical material in a sample is bonded to the fixed protein or produces a product. At this time, the bonding material or the product is labeled with a fluorescent material, and the nanowire light sensor which includes a nanowire appropriate for a fluorescence wavelength of the fluorescent material is used to measure fluorescence being generated. Accordingly, a protein chip can be provided which can detect a reaction between an enzyme and a substrate, a bond between a receptor (synthetic or biological receptor) and a bonding material, a bond between protein such as an antigen-antibody reaction, a bond between protein and a gene (DNA or RNA) and a bond between genes (DNA or RNA).

In addition, a protein chip can be provided for measuring chemiluminescence by bonding a luminous enzyme to the bonding material and the product and injecting a luminous material thereinto and by using the nanowire light sensor which includes a nanowire appropriate for a luminescence wavelength of the luminous material. The application of the nanowire light sensor is possible when the nanowire light sensors are integrated to have an array form like the nucleic acid detection chip. That is, the protein chip can be used in all tests for determining whether a reaction occurs or not and the reaction amount through the measurement of fluorescence, such as a reaction between an enzyme and protein, a reaction between (synthetic or biological receptor) and a bonding material, a bond between protein such as an antigen-antibody reaction, a bonding reaction between protein and a gene (DNA or RNA).

In the immunoassay kit including such nucleic detection chip and the protein chip, a micro array type protein chip or a protein chip for a single test can be implemented using the nanowire light sensor. In case of the micro array type, a plurality of nanowire light sensors are connected through a multiplexer such that signals of respective light sensors are sequentially processed or through analog switching, signals of respective light sensors are processed at the same time.

Above-described measurements of fluorescence and luminescence are performed by fixing the nanowire light sensor adjacently to part where fluorescence and luminescence are generated. Such a fixing method can be used without great modification in the existing rapid test kit, protein chip and nucleic detection chip being manufactured.

In addition, a method for generating fluorescence and luminescence on a nanowire can be used to reduce loss of the amount of light producing a signal and for the rapid measurement. That is, as described so far, direct fixation of a reactant, which is fixed to a solid, to the nanowire can be realized. Such a method for fixing the reactant to the nanowire comprises coating the nanowire with a chemical linkage material (linker) between the nanowire and the material and fixing the reactant to the linkage material. A material which does not absorb light of a corresponding wavelength in order not to prevent generated fluorescence or luminescence from reaching the nanowire is selected as the chemical linkage material used in the nanowire coating. In addition, the chemical linkage material should be coated closely to a single layer which is thin and even in order not to spatially prevent a bond of a ligand material. In case of the nanowire formed of a metallic oxide, a chemical linkage material film can be formed on the nanowire by processing a thiol or organic silane derivative that carboxyl, an amine group, an epoxide group, sulfone acid, or the like is bonded to the end of a pure carbon chain or of a carbon chain having a chemical functional group like ester at its center so that the chemical linkage material film can be formed on the nanowire. The organic silane derivative may be an anhydrous trixtoxy or a trimetoxy organic silane derivative.

In addition, in case of a nanowire formed of a metallic oxide, pure metal, minerals or the like, after a metal film is coated by a process such as a CVD (chemical vapor deposition) method, a thiol derivative that an amine group, carboxyl, an epoxide group, sulfone acid, or the like is bonded to the end of a carbon chain which has various lengths is processed to thereby form the chemical linkage material film on the nanowire.

Thereafter, as for a bond of the reactant, when the end of the chemical linkage material is carboxyl, N-(3-dimethylaminopropyl)-N-ethylcarbodiimide (EDAC) and N-hydroxysuccinimide (NHS) or the like is used. In case of the amine group, glutaraldehyde or the like is used. In case of the epoxide group and sulfone acid, the reactant is incubated for a predetermined time to induce a bond.

By using the method for directly bonding the reactant on the nanowire, a reaction area used to bond the reactant increases according to growth density of the nanowire in comparison to a plane to which the nanowire is fixed, and thereby, a greatly amplified sensor signal can be obtained from an antigen-antibody bonding reaction, a reaction by an enzyme, a bonding reaction using DNA and RNA, and the like.

Hereinafter, examples in which a nanowire light sensor is manufactured, the nanowire light sensor is compounded with a chemiluminescence material to thereby manufacture a rapid test kit, and the performance is tested will be described.

### [Example]

### Example 1

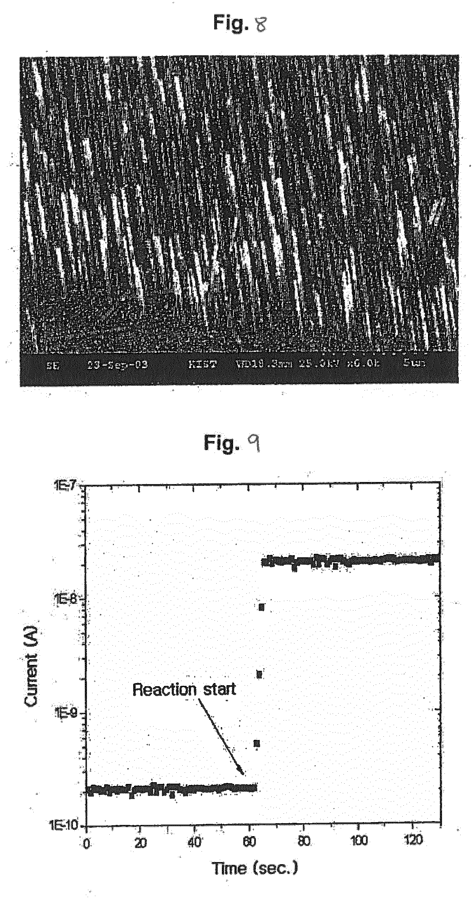

By depositing Au which was as thick as 2 nm on one surface of a silicon substrate by a sputtering deposition method and providing InCl₃ and NH₃ on the substrate at 500°C by CVD, a InN nanowire was grown. Figure 8 shows InN nanowire grown on the substrate. The nanowire was separated from the substrate by putting the substrate in Isopropyl alcohol and applying ultrasonic waves for 10 seconds. The nanowire was aligned and located between Ti/Au electrodes by dispersing the solution on the substrate where the electrodes are located and applying potential difference of 20 V therebetween. Then, by heating (500°C, 30 seconds) or electron-beam irradiation, the electrodes were connected with the nanowire to thereby manufacture a nanowire sensor. After checking electric connection, as shown in Figure 6, the manufactured nanowire sensor was located to be attached to a signal line and a check line where chemiluminescence occurs. At this time, a luminol derivative was used as a detection target substrate. At this time, a change of an electric current which occurs in a state that the chemiluminescence was being generated is shown in Figure 9. According to the current change, a result of such detection with respect to a target material can be checked.

### Example 2

By depositing Au which was as thick as 2 m on one surface of a sapphire substrate by a sputtering deposition method and providing TMG (trimethylgallium), NH3 and a small amount of Cp2Mg on the substrate at 700°C by CVD, a GaN nanowire doped with Mg was grown. A test kit was constructed using the grown nanowire by a method identical to Example 1. At this time, an acrydinum compound was used as a detection target substrate and a detection result with respect to a target material was checked by a change of an electric current which occurs on the nanowire.

### Example 3

By depositing Au which was as thick as 2 nm on one surface of a sapphire substrate by a sputtering deposition method, locating In metal on the substrate, and providing NH₃, a InN nanowire was grown. A test kit was constructed using a grown nanowire by a method identical to Example 1. At this time, photoprotein was used as a detection target substrate and a detection result with respect to a target material was checked by a change of an electric current which occurs on the nanowire.

### Example 4

By depositing Ni which was as thick as 2 nm on one surface of a sapphire substrate by a sputtering deposition method, and providing TMG (trimethylgallium) and NH3 on the substrate at 700°C by CVD, a GaN nanowire was grown. Then, a nanowire having radial heterostructure where the core is GaN and the sheath is ZnO was composed by continuously providing DEZ (dethyl zinc) and O₂. A test kit was constructed using the grown nanowire by a method identical to Example 1. At this time, an acrydinum compound was used as a detection target substrate and a detection result with respect to a target material was checked by a change of an electric current which occurs on the nanowire.

### Example 5

By depositing Au which was as thick as 2nm on one surface of a sapphire substrate by a sputtering deposition method, and providing DEZ (dethyl zinc) and O₂ on the substrate at 700°C by CVD, a ZnO nanowire was grown. Then, GaN was deposited on a surface by continuously providing TMG (trimethylgallium) and NH₃. Then, ZnO core was removed by processing the nanowire in a HCL solution for 12 hours and therefore a GaN nanotube was composed. A kit was constructed using the composed nanotube by a method identical to Example 1. At this time, an acrydinum compound was used as a detection target substrate and a detection result with respect to a target material was checked by a change of an electric current which occurs on the nanowire.

### Example 6

By depositing Ni which was as thick as 2nm on one surface of a sapphire substrate by a sputtering deposition method, mixing Ga and In metal by a mass ratio of 3: 1 and locating the mixed Ga and In, and providing NH₃, a GA₁₋ₓInₓN nanowire was grown. A test was constructed using a grown nanowire by a method identical to Example 1. At this time, photoprotein was used as a detection target substrate and a detection result with respect to a target material was checked by a change of an electric current which occurs on the nanowire.

### Example 7

After fixing CA 125 (NatuTec GmbH, Germany), a dark labeling material, to a micro plate, a CA 125 antibody (NatuTec GmbH, Germany) to which HRP was bonded in various concentrations. Then, after applying a luminous substrate solvent containing luminol, the chemiluminescence amount was measured using the InN nanowire light sensor obtained in Example 1. As a test result, the InN nanowire light sensor showed a quantitative reaction according to concentrations of bonded HRP and antibody. In order to check a measurement result, chemiluminescence was measured using the existing luminescence and fluorescence measuring device (LS45, Perkin-Elmer) at the same time. A test result showed that the light sensor manufactured with the nanowire appropriate for a chemiluminescence wavelength was appropriate for the quantitative measurement of chemiluminescence.

### Example 8

A hepatitis B antibody to which fluorescein was bonded was bonded to a glass substrate to which a hepatitis B antigen was bonded in various concentrations. A laser of 495nm, exciting wavelength of fluorescein, was irradiated and simultaneously fluorescence occurring at the 90 ° angle to the laser was measured by the InN nanowire light sensor. In order to check a measurement result, fluorescence was measured using the existing luminescence and fluorescence measuring device (LS45, Perkin-Elmer). According to a test result, the GaP nanowire light sensor showed a quantitative signal according to the amount of the bonded fluorescein. The test result shows that the light sensor manufactured with the nanowire appropriate for a fluorescence wavelength can quantitatively measure fluorescence.

As described so far, a light sensor including a nanowire and a kit show improved resolution by measuring chemiluminescence and chemifluorescence using the nanowire light sensor. Since the kit uses the nanowire light sensor, miniaturization of the kit is possible, the unit cost of production is low to be used as disposables, and the kit can be used on the spot without separate external equipment or blocking of light, so that the can be applied as a kit for immunoassay, a nucleic acid chip and a protein chip. In addition to this, the light sensor using the nanowire can be widely used in all reactions where quantitative analysis using chemiluminescence or chemifluorescence is performed.

## Claims

1. A method for manufacturing a nanowire light sensor, comprising:
growing a semiconductor nanowire with diameter in the range of one nanometer to 100 nanometers on a substrate and then separating the grown semiconductor nanowire;
locating two conductive metal thin film electrodes on a nonconducting substrate, in which a distance between the two electrodes is shorter than the length of the semiconductor nanowire;
locating the semiconductor nanowire between the two electrodes so as to connect the two electrodes by dispersing the obtained semiconductor nanowire between the two electrodes and
supplying voltages; and **characterized by**
electrically connecting the electrodes and the semiconductor nanowire by electron-beam irradiation.

2. The method of claim 1, wherein silicon or sapphire is used as the substrate, and a material selected from the group Au, Ti, Pt, Pd, TiN and alloys of two or more of them as the electrodes.

3. The method of claim 1, wherein an appropriate cursor is provided and grown such that the nanowire is formed of one or more materials selected from the group consisting of ZnO, SnO₂, CdSe, GaN, CdS, InP, GaP, GaAs, AlAs, InN, Si, Ge, and SiC.

4. The method of claim 3, wherein the nanowire is formed of two or more materials, so that a light sensitivity bandgap is controlled into a desired range, or wherein the nanowire is doped with appropriate impurities, so that a light sensitivity bandgap is controlled into a desired range.

5. The method of claim 3, wherein the nanowire has radial heterostructure including core part and sheath part which are formed of materials different from each other, so that a light sensitivity bandgap is controlled into a desired range, or wherein the nanowire has longitudinal heterostructure by alternately depositing different materials, so that a light sensitivity bandgap is controlled into a desired range.

6. The method of claim 3, wherein the nanowire has a tubular shape of which center portion is removed, so that a light sensitivity bandgap is controlled into a desired range, or wherein the nanowire comprises two or more materials being in a solid solution with respect to each other, so that a light sensitivity bandgap is controlled into a desired range.

## Patentansprüche

1. Verfahren zur Herstellung eines Nanodraht-Lichtsensors, umfassend:
Züchten eines Halbleiter-Nanodrahts mit einem Durchmesser im Bereich von einem Nanometer bis 100 Nanometer auf einem Substrat und anschließend Ablösen des gezüchteten Halbleiter-Nanodrahts;
Platzieren zweier leitender metallischer Dünnschichtelektroden auf einem nichtleitenden Substrat, wobei die Entfernung zwischen den beiden Elektroden kürzer als die Länge des Halbleiter-Nanodrahts ist;
Platzieren des Halbleiter-Nanodrahts zwischen den beiden Elektroden zum Verbinden der beiden Elektroden durch Verteilen des erzeugten Halbleiter-Nanodrahts zwischen den beiden Elektroden und
Zuführen von Spannungen; und **dadurch gekennzeichnet, dass**
die Elektroden und der Halbleiter-Nanodraht durch Elektronenstrahlbestrahlung miteinander elektrisch verbunden werden.

2. Verfahren nach Anspruch 1, wobei Silizium oder Saphir als das Substrat verwendet wird und ein aus der Gruppe bestehend aus Au, Ti, Pt, Pd, TiN und Legierungen aus zwei oder mehreren der genannten Materialien ausgewähltes Material als die Elektroden verwendet wird.

3. Verfahren nach Anspruch 1, wobei ein geeigneter Cursor bereitgestellt und gezüchtet wird, sodass der Nanodraht aus einem oder mehreren, aus der Gruppe bestehend aus ZnO, SnO₂, CdSe, GaN, CdS, InP, GaP, GaAs, AlAs, InN, Si, Ge und SiC ausgewählten Materialien ausgebildet wird.

4. Verfahren nach Anspruch 3, wobei der Nanodraht aus zwei oder mehreren Materialien ausgebildet wird, sodass eine Lichtempfindlichkeits-Bandlücke in einen gewünschten Bereich gelenkt wird, oder wobei der Nanodraht mit geeigneten Verunreinigungen dotiert wird, sodass eine Lichtempfindlichkeits-Bandlücke in einen gewünschten Bereich gelenkt wird.

5. Verfahren nach Anspruch 3, wobei der Nanodraht eine radiale Heterostruktur umfassend einen Kernteil und einen Schalenteil aufweist, die aus voneinander unterschiedlichen Materialien ausgebildet sind, sodass eine-Lichtempfindlichkeits-Bandlücke in einen gewünschten Bereich gelenkt wird, oder wobei der Nanodraht eine durch abwechselndes Ablagern unterschiedlicher Materialien geschaffene longitudinale Heterostruktur aufweist, sodass eine Lichtempfindlichkeits-Bandlücke in einen gewünschten Bereich gelenkt wird.

6. Verfahren nach Anspruch 3, wobei der Nanodraht eine rohrförmige Form aufweist, bei welcher der zentrale Abschnitt entfernt wurde, sodass eine Lichtempfindlichkeits-Bandlücke in einen gewünschten Bereich gelenkt wird, oder wobei der Nanodraht zwei oder mehr Materialien umfasst, die in Bezug aufeinander in einer festen Lösung vorliegen, sodass eine Lichtempfindlichkeits-Bandlücke in einen gewünschten Bereich gelenkt wird.

## Revendications

1. Procédé de fabrication d'un capteur de lumière de nanofil, comprenant :
cultiver un nanofil semi-conducteur d'un diamètre de l'ordre d'un nanomètre à 100 nanomètres sur un substrat, et puis séparer le nanofil semi-conducteur cultivé ;
positionner deux électrodes à couche mince étant conductrices et métalliques sur un substrat non conducteur, dans lequel une distance entre les deux électrodes est plus courte que la longueur du nanofil semi-conducteur ;
positionner le nanofil semi-conducteur entre les deux électrodes de manière à connecter les deux électrodes en dispersant le nanofil semi-conducteur obtenu entre les deux électrodes et
fournir des tensions ; et **caractérisé par**
connecter électriquement les électrodes et le nanofil semi-conducteur par irradiation par faisceau d'électrons.

2. Procédé selon la revendication 1, dans lequel le silicium ou le saphir est utilisé comme substrat, et un matériau choisi dans le groupe consistant en Au, Ti, Pt, Pd, TiN et des alliages de deux ou plusieurs d'entre eux est utilisé comme électrodes.

3. Procédé selon la revendication 1, dans lequel un curseur approprié est prévu et cultivé de sorte que le nanofil est formé d'un ou plusieurs matériaux choisis dans le groupe consistant en ZnO, SnO₂, CdSe, GaN, CdS, InP, GaP, GaAs, AlAs, InN, Si, Ge et SiC.

4. Procédé selon la revendication 3, dans lequel le nanofil est formé de deux matériaux ou plus, de sorte qu'une bande interdite de sensibilité à la lumière est guidée dans une zone souhaitée, ou dans lequel le nanofil est dopé avec des impuretés appropriées, de sorte qu'une bande interdite de sensibilité à la lumière est guidée dans une zone souhaitée.

5. Procédé selon la revendication 3, dans lequel le nanofil présente une hétérostructure radiale comprenant une partie de noyau et une partie d'enveloppe qui sont formées des matériaux différents l'un de l'autre, de sorte qu'une bande interdite de sensibilité à la lumière est guidée dans une zone souhaitée, ou dans lequel le nanofil comprend une hétérostructure longitudinale crée par dépôt alterné des matériaux différents, de sorte qu'une bande interdite de sensibilité à la lumière est guidée dans une zone souhaitée.

6. Procédé selon la revendication 3, dans lequel le nanofil a une forme tubulaire dont la partie centrale est enlevée, de sorte qu'une bande interdite de sensibilité à la lumière est guidée dans une zone souhaitée, ou dans lequel le nanofil comprend deux ou plusieurs matériaux se trouvant dans une solution solide l'un par rapport à l'autre, de sorte qu'une bande interdite de sensibilité à la lumière est guidée dans une zone souhaitée.
